(19) **Europäisches Patentamt / European Patent Office / Office européen des brevets**

(11) **EP 4 234 096 B1**

(12) **EUROPEAN PATENT SPECIFICATION**

(45) Date of publication and mention
of the grant of the patent:
**22.07.2026 Bulletin 2026/30**

(21) Application number: **23178445.5**

(22) Date of filing: **24.07.2019**

(51) International Patent Classification (IPC):
**B65D 83/32** *(2006.01)* **B65D 83/68** *(2025.01)*
**B05B 7/28** *(2006.01)* **B65D 83/62** *(2006.01)*
**B65D 83/66** *(2025.01)* B05B 7/04 *(2006.01)*

(52) Cooperative Patent Classification (CPC):
**B65D 83/625; B05B 7/28; B65D 83/32;
B65D 83/58; B65D 83/66**

(54) **BAG ON VALVE TECHNOLOGY**

BEUTEL-AUF-VENTIL-TECHNOLOGIE

TECHNOLOGIE DE POCHE SUR VANNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.07.2018 GB 201812298**

(43) Date of publication of application:
**30.08.2023 Bulletin 2023/35**

(62) Document number(s) of the earlier application(s) in
accordance with Art. 76 EPC:
**19769236.1 / 3 830 002**

(73) Proprietor: **SIMPLY BREATHE HOLDINGS LTD
Beverly HU17 0QT (GB)**

(72) Inventors:
• **BARRATT, Joe Michael
Beverley HU17 0FT (GB)**

• **SYGROVE, Matthew James
Halifax, Ogden HX2 9NX (GB)**
• **SCHILLER, Dominic Christopher
Merseyside, CH41 5AR (GB)**
• **RYAN, Thomas Anthony
Tarporley CW6 0PD (GB)**

(74) Representative: **Schiller, Dominic Christopher
Equipped 4 (IP) Limited
47 Hamilton Square
Birkenhead, Merseyside CH41 5AR (GB)**

(56) References cited:
EP-A1- 2 327 921    WO-A1-90/05580
DE-A1- 1 817 840    DE-A1- 1 817 899
FR-A- 1 567 923    GB-A- 2 510 400
US-A- 3 326 469    US-A- 3 583 604
US-A- 3 796 352    US-A- 3 896 970
US-A1- 2006 049 278    US-A1- 2008 116 228

## Description

This invention relates to improvements in a delivery technology referred to in the industry as bag-on-valve (BOV) technology and to a dispenser for use with novel valve assemblies. The dispenser, and method of delivery, utilise a dispensing carrier gas, typically an aerial gas, which is adsorbed on, typically, activated carbon.

## BACKGROUND TO THE INVENTION AND PRIOR ART

The global bag on valve market was worth US$356.5 m in 2016 and is growing due to the rising awareness about its cost-effectiveness amongst consumers and manufacturers. Consumers are showing a strong inclination toward bag-on-valve technology as a packaging solution as it minimizes product wastage, thereby ensuring value for money. Thus, this technology is being used for several high-end products. Yet another noticeable benefit is the environmental benefits this technology brings, due to its airless bags and the method of packaging that separates the product and the propellant.

For the manufacturer, the preference for bag-on-valve promises a longer shelf life for oxygen sensitive products that contain fewer or no preservatives. Furthermore, various types of viscous and liquid products can be packaged using bag-on-valve technology, irrespective of the fact that they may be water or solvent-based. A growing number of manufacturers are also investing in this technology as the absence of propellant in the product reduces the risk of explosion or fire. Also, the efficient filling process is an additional advantage.

However, there are limitations and challenges resulting from the propellants used and the pressure drop during operation which, for example, preclude the complete emptying of the bag.

Legislation has been brought in to remove the high Global Warming Potential (GWP) of liquified gas propellants from aerosols.

The dispenser of the invention preferably uses aerial gases, particularly $CO_2$ which means it has no GWP. This is because whilst the GWP of carbon dioxide is 1, it is derived from the atmosphere, and so the nett effect of using it is zero.

This contrasts with the new liquified gas propellants (such as HFO-1234ze) which have low GWP because they break down in the atmosphere within a short period of time. However, the breakdown products are pollutants.

Fluorocarbon-based propellants are subject to a never-ending cycle of regulatory change. However, aerial gases are exempt from legislation such as the REACH regulation.

Over the last 12 years in the UK there has been a reduction in VOC emissions of 30 %. However, in that time, the emissions from aerosols has increased by 10 % which is roughly in line with the increase in population size over this period. However, since 2005 the emissions from aerosols have grown from 4.7% to 7.9% (2017). The Government wants to reduce total VOC emissions by 38 % by 2030 and BAMA (British Aerosols Manufacturing Association) want to develop plans to reduce VOC emissions in aerosols whilst maintaining the high levels of product performance, consumer acceptance and safety.

A reason for the continued use of fluorocarbon-based propellants is compressed aerial gases cannot be stored in a sufficient quantity to enable ingredients to be fully discharged from a bag on valve aerosol can.

Applicants use of activated carbon to enhance the gas storage volume enables the contents of normal sized pouches to be discharged in full.

Further, by avoiding the use of solvents and liquified gas propellants solvent abuse is mitigated.

Aerosols that use water as a solvent and compressed air propellants have poor (low force) performance and produce a wet spray with a low plume. In contrast the dispensers described herein produce an almost dry spray with good force and plume.

Additionally, whilst traditional aerosols are subject to dip-tube inversion, causing the release of excess propellant, the dispensers described herein avoid inversion problems.

Also, regular bag-on-valve technology does not enable the active ingredient in the bag to aerosolise whereas the dispensers described herein enable aerosolization (dispersal into an aerosol form).

Products available in the global market are aerosol BOV, standard BOV, and non-spray/low pressure BOV. Of these, the aerosol BOV is expected to acquire over 60% of the global market by the end of 2024.

The top four players are AptarGroup, Inc., Coster Tecnologie Speciali S.p.A, Toyo & Deutsche Aerosol Gmbh, and Summit Packaging System, Inc. These companies collectively held a share of about 39% in the global market in 2015.

The valve or valve assembly comprises either a male or female valve which is connected or crimped to a dispenser container or canister which is made of aluminium, tin plate, steel or plastics. Typical dispenser container capacity falls into one of the following size categories: below 30ml, 30ml - 100ml, 100ml - 275ml, 275ml - 500ml and above 500ml.

Typical applications include applications for the following product types: Cosmetics & Personal Care products, e.g. deodorants and antiperspirants, Pharmaceutical products, Home Care products, e.g. air fresheners, cleaning preparations, Food & Beverage products e.g. cream and cheese, and Automotive & Industrial products e.g. paints.

These traditional bag-on-valve dispensers, like aerosol dispensers, normally contain one of two types of propellant.

i) Liquefied gas propellants, which are primarily hydrocarbon based (e.g. propane/n-butane/iso-butane blends); or

ii) Hydrofluorocarbon based, (e.g. HFC-134a, -152a or HFO-1234ze).

The negative issues surrounding hydrocarbon propellants are well known, since these compounds are highly flammable, volatile organic compounds (VOC's) that are the subject of inhalation abuse and contribute to poor indoor air quality.

The hydrofluorocarbons are also replete with problems in aerosol applications, and HFC-134a, for example, has been recently legislatively phased out from use in many applications owing to its intrinsically high GWP.

Two condensed gas compounds that meet the new EU F-Gas Regulations for GWP<150 include:

HFC-152a (1,1-difluoroethane); and

HFO-1234ze (1,3,3,3-tetrafluoroprop-2-ene).

Unfortunately, HFC-152a (GWP~120) is designated as highly flammable, and HFO-1234ze (GWP~6) is conceded to be flammable above 28 °C. It is also oftentimes prohibitively expensive.

Of course, where there is combustion of HFCs or HFOs there is also the release of hydrogen fluoride which is both very toxic and corrosive. Additionally, there is increased reporting of fluorinated hydrocarbon (HFC) abuse, particularly with HFC-152a, amongst young adults resulting in occasional deaths. It is too soon to report on the abuse of HFO-1234ze but there is every reason to assume that it will provide similar euphoric/asphyxiant properties to those of the HFCs. Finally, although there is only a small GWP contribution, the environmental breakdown of HFO-1234ze produces fluoroacetic acids which are toxic to plant and aquatic life. One of the atmospheric breakdown products of HFC-152a is carbonyl difluoride ($COF_2$) which hydrolyses in the lower atmosphere to give hydrogen fluoride.

Because of this it is desirable to avoid the use of such gases and to use compressed aerial gases where possible.

The development of a bag- and frit-on- valve assembly, as outlined herein, has many advantages and allows the use of non-harmful gases, e.g. aerial gases for dispensing a wide range of ingredients in a wide range of applications.

The benefits of, for example, aerial gases, such as air, nitrogen, oxygen, argon or carbon dioxide, arise from the fact they are cheap, readily available, and have low toxicity and are without risk of phase-out. These gases are also not amenable to regulation such as the REACH Regulation.

Unfortunately, aerial gases cannot be easily condensed without refrigeration, or the use of extreme pressures (below the respective critical temperatures) to provide gas in sufficient quantity for aerosol applications. Compression of these gases into dispenser containers is easily possible although the maximum, permitted pressure is limited such that the contents pressure does not exceed 15 barg when the canister and its contents are raised to the test temperature of 50 °C for 3 minutes, according to the Aerosols Directive. This restriction means that a canister must not be filled much above 12 barg at room temperature. Under such conditions, upon actuation, the pressure drops rapidly as the canisters' contents are discharged, and the overall gas volume delivery is small giving rise to a small number of delivered applications and poor customer perception.

By way of a non-limiting example, a standard air freshener employing a liquefied gas usually contains an ingredient (product concentrate) and a solvent in addition to the liquefied propellant; either hydrocarbon or HFC with all of the disadvantages as already described. Additionally, there is the possibility of product misuse resulting from dip-tube inversion giving a disproportionate loss of propellant. A standard, compressed air-based, air freshener, might thus contain 5 % ethanol in water compressed with air in addition to the dissolved fragrance concentrate. Such devices tend to deliver a short, wet spray and although this system does not contain any liquefied gas propellant, it still contains solvent and exhibits poor performance.

In view of the shortcomings described for both liquefied gas-containing aerosols and for compressed gas-containing aerosols, it appears that an aerosol canister containing neither liquefied gas propellant nor solvent would be advantageous.

Whilst bag-on-valve technology enables the active product to be separated from the propellant (typically compressed air or nitrogen, or a condensed liquified gas), to maintain complete integrity of the product so that only pure product is dispensed, these standard bag-on-valves do not aerosolise because they do not release the propellant, but they can atomize the liquid products when sprayed.

In consequence their use is limited to, for example, the dispensation of liquids, including viscous liquids, solutions, lotions, creams, pharmaceutical preparations, gels, olive oil and other food products, such as processed cheese. As a consequence of being enclosed in a bag, the active ingredient is protected from oxygen contact which might otherwise cause the product to spoil, and it is protected from contact with the propellant because the propellant is filled into the space occupied between the bag and the can.

Prior art, separate of traditional bag-on-valve dispensers, include art relating to adsorbent carbon technology such as Applicants own UK application no GB1703286.3 and WO 2014/037086, in which an aerial propellant gas is adsorbed onto activated carbon contained within a canister (in the space between the bag and the canister) which enables a more even dispensation of the contents of the bag compared to a compressed gas alone. Like the traditional bag-on-valve arrangements, no gas is discharged from the canister.

DE1817899 discloses a dispenser comprising a liquified gas propellant. A liquid to be atomised is contained in a bag located in the container and a double valve allows for a fluid flow circuit in which the liquid is atomised as it is sucked up by means of a venturi tube.

DE1817840A1 discloses a dispenser comprising a container that houses a cartridge comprising a liquified propellant and a bag comprising a liquid ingredient. The dispenser further comprising a valve and a Venturi nozzle. The propellant and the liquid ingredient mix in the Venturi nozzle, when the valve is actuated.

## BRIEF SUMMARY OF THE DISCLOSURE

In accordance with a first aspect of the present inventions there is provided a dispenser (20) comprising a dispenser container (90) and a valve assembly (10) comprising either a male or female valve connected or crimped to the dispenser container wherein:

a) the dispenser container (90) is partially filled with activated carbon (130) or another adsorbent and a dispensing carrier gas (140),

b) an ingredient (100) is contained in an ingredient containing reservoir (110/150), and

c) the valve assembly (10) comprises a component comprising a dip tube (80), an upper end of which is in operative communication with an actuator spray nozzle (200), and a lower end of which divides into first and second fitments (182; 184) which receive respectively first and second tubes (82; 84); the first tube (82), seated within the dispenser, is connected to the ingredient (100) containing reservoir (110/150), allowing the ingredient to be dispensed on actuation of the valve, and the second tube (84) comprises a frit or filter (120) to prevent the activated carbon (130) or another adsorbent passing into the second tube (84) when the pressurised dispensing carrier gas (140) is released;

such that on actuation, the dispensing carrier gas (140) is released together with the ingredient (100), and the ingredient (100) and dispensing carrier gas (140) mix as they pass through the valve assembly (10) to exit the dispenser container via the actuator spray nozzle (200).

In one embodiment the dip tube of the valve assembly divides into the first and second tubes which are seated within the dispenser container. The first tube is connected to the ingredient containing reservoir allowing the ingredient to be dispensed on actuation of the valve. The second tube comprises a frit or filter to prevent activated carbon passing into the tube when the pressurised dispensing carrier gas is released.

Preferably the ingredient containing reservoir is a bag or pouch.

The dispenser may comprise three or more tubes and at least two ingredient containing reservoirs comprising different ingredients.

The dispenser may further comprise a metering device.

The dispenser may further comprise a spacer.

The dispenser is preferably filled with a dispensing gas which is nitrous oxide or an aerial gas, such as air, nitrogen, oxygen, carbon dioxide or argon.

Most preferably the aerial gas is carbon dioxide since it is the gas that is most effectively absorbed by the activated carbon.

A benefit of the invention is that it is able to dispense an ingredient absent of a liquified propellant and/or a solvent.

The active ingredient may include any ingredient used in the cosmetics & personal care, pharmaceutical, home care, food & beverage and automotive & industrial sectors, including particularly, but not exclusively, a fragrance, flavour, pheromone, pesticide, medicinal, nutraceutical or pharmaceutical ingredient.

In the case of medicines and pharmaceuticals it is essential that a constant dose is delivered, and thus the dispenser is further adapted to deliver a metered dose and may additionally comprise a spacer.

In accordance with a second aspect of the present invention there is provided a method of delivering an ingredient (100) from a dispenser (20) of the first aspect of the present invention wherein the dispensing carrier gas (140) is released together with the ingredient (100), and the ingredient (100) and dispensing carrier gas (140) mix as they pass through the valve assembly (10) to exit the dispenser container via the actuator spray nozzle (200).

Preferably, the ingredient is released from an ingredient container under pressure together with a dispensing carrier gas which is also released on actuation of a valve assembly, which carrier dispensing gas travels along the second tube into the ingredient container and carries the ingredient along the first tube where they mix in the valve assembly before exiting the dispenser via an actuator spray nozzle to an environment or subject.

In one embodiment mixing effectively commences in the valve assembly, whereas in another embodiment mixing commences as the carrier gas passes through the ingredient container.

In a preferred embodiment the ingredient containing reservoir is a bag or pouch.

In all embodiments the valve assembly further comprises an actuator.

In yet another embodiment the valve assembly comprises three or more tubes and at least two ingredient containing reservoirs comprising different ingredients.

A preferred ingredient is a fragrance and the product an air freshener. Other preferred ingredients include deodorisers and antiperspirants, hairsprays, polish cleaners and emulsions.

In a favoured embodiment, particularly for the delivery of pharmaceutical or medicinal ingredients, the valve assembly further comprising a metering device and optionally a spacer.

The activated carbon is added in an amount that allows for the ingredient containing bag(s) to be filler. Typically, the amount will be from 15 -65% by volume, more typically 25 to 45% by volume, of the dispensing container depending on the size of the bag(s).

Also, in order to control spray performance a pressure of between 4 and 10 barg, and more preferably 6 to 8 barg is preferred.

Flow of the dispensing gas and ingredient may also be controlled by fitting a reducer in the actuator or by judicious selection of a valve restrictor orifice. Indeed, it may be desirable to use different diameter tubes/ valve orifices to control the ratio of ingredient: dispensing gas flow. Generally, ensuring a greater flow of the dispensing gas to liquid will result in a drier plume.

Obviously, the discharge rate will vary with the ingredient but for many consumer goods it is desirable to have a discharge rate in the range 0.2 to 0.5g/s.

In some cases, it may also be desirable to include a small proportion of an entraining agent with the dispensing gas.

## BRIEF DESCRIPTION OF THE DRAWINGS

Embodiments of the invention are further described hereinafter with reference to the accompanying drawings, in which:

Fig 1A is an exploded view of a prior art male (single) bag on valve assembly;

Fig 1B is a cross sectional view of the assembled valve assembly of Fig 1A;

Fig 2A is an exploded view of a first embodiment of a valve assembly of the present invention;

Fig 2B is an exploded view of a first embodiment of a dispenser comprising the valve assembly of Fig 2A;

Fig 2C is a side elevation of the assembled dispenser of Fig 2B;

Fig 2D is a cross sectional view of the dispenser of Fig 2B;

Fig 2E is a detailed view of the encircled area of Fig 2D;

Fig 3A is an exploded view of a dispenser comprising an alternative valve assembly which falls outside the scope of the claimed invention; and

Fig 3B is a cross sectional view of the dispenser of Fig 3A.

## DETAILED DESCRIPTION

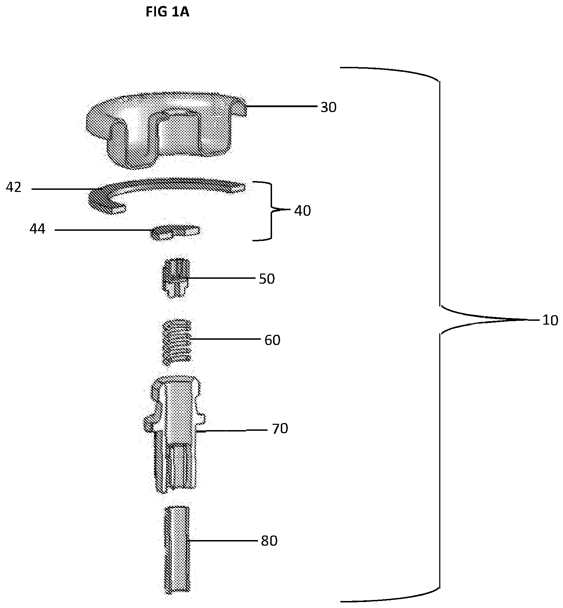

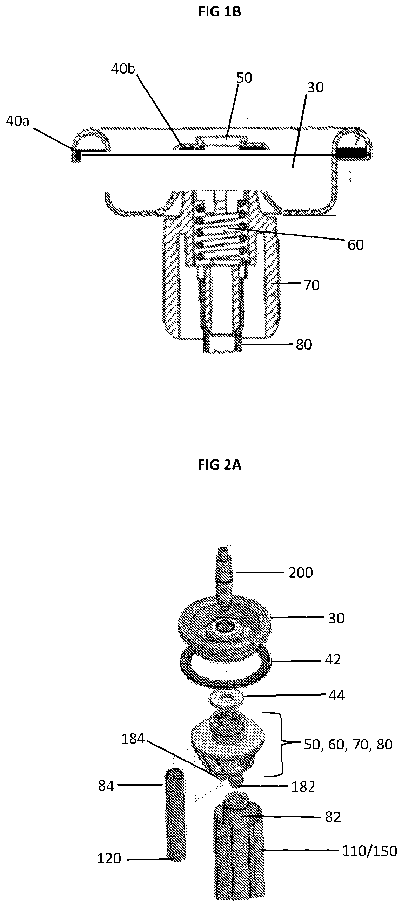

Referring to Fig 1 a typical bag on valve assembly (10) comprises:

i) a mounting cup (30);

ii) an outer (42) and inner (44) gasket (40);

iii) a valve seat (50);

iv) a spring (60);

v) a housing (70); and

vi) a dip tube (80) with a fitment, such as a rib, to which a bag (not shown) is attached.

Various actuators (200) may be connected to the valve assembly (10) which may be a male valve (as illustrated) or a female valve.

In a variation to the single bag arrangement two companies, Lindal Group (Bivalve) and Toyo Aerosol industry (Dual) have developed a dispensing system in which two bags are filled, allowing two different products to be dispensed, either as separate products, or more typically as a single product, with mixing occurring in the valve assembly. In the latter case the valve assembly has a dip tube (80) which splits / bifurcates into two, each with fitments for connecting a bag thereto. The bags are typically 3 layer, or 4 layer, pouches made respectively of polyacrylate/ aluminium/ polypropylene or polyethylene (PA/ ALU/ PP or PE) or polyethylene terephthalate/ aluminium/orientated polyamide/ polypropylene or polyethylene (PET/ ALU/ OPA/ PP or PE).

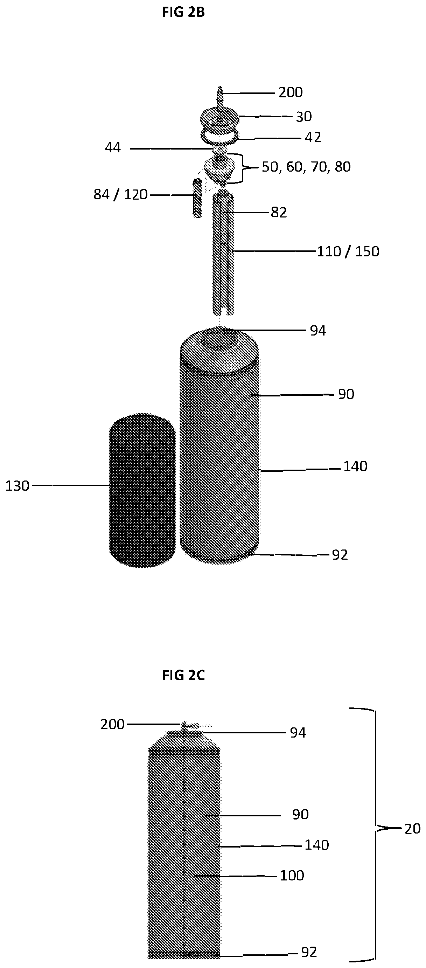

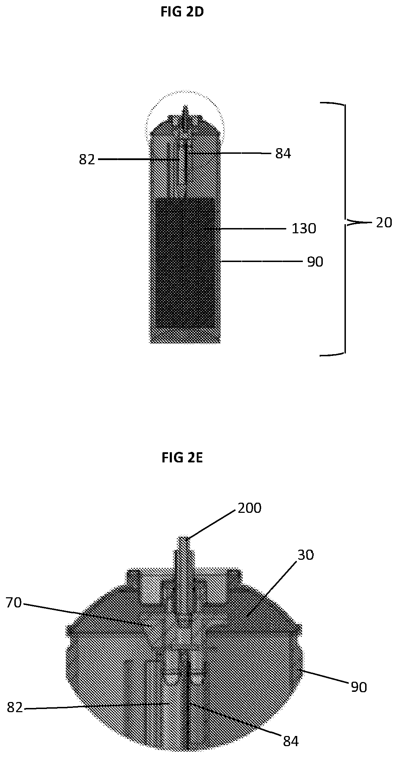

In contrast to the prior art, the valve assembly (10) according to the first aspect of the invention (as best illustrated in Figs 2A and 2B, has a mounting cup (30), a pair of gaskets (42 and 44), a valve seat (50), spring (60) and housing (70), with a dip tube (80) which divides, at its lower end, to receive two tubes (82; 84) on respective fitments (182; 184). An ingredient (100) containing reservoir (110) or bag or pouch (150) is connected to a first tube (82) and a frit or filter (120) is connected to a second tube (84), and acts to prevent fine particles of activated carbon being dispensed. Both tubes extend into dispenser container (90), which is filled with a dispensing carrier gas (140), typically carbon dioxide, which is adsorbed by activated carbon (130) which fills or partially fills the dispenser container (90). On actuation, the dispensing carrier gas (140) is released together with the ingredient (100) stored in the bag (150), and the ingredient (100) and carrier gas (140) mix as they pass through the valve assembly (10) to exit the dispenser container via the actuator spray nozzle (200).

The dispenser (20) illustrated in Fig 2D, comprises a dispenser container or cannister (90) (Fig 2B) which is filled or partially filled with activated carbon (130) and the valve assembly (10) is crimped, or otherwise sealed, to close the opening (94) (Fig 2B) of the dispensing cannister (90). The dispenser (20) may be charged with a dispensing carrier gas (140) before or after crimping or otherwise sealing, as disclosed in, for example UK application no GB 1703286.3. Similarly, the bag or pouch (150) may be filled with its ingredients (100) before or after crimping.

The filled dispenser (20) is substantially as illustrated in Figs 2C and 2D.

The invention enables, for example, essential oils/fragrances to be rapidly mixed by vaporisation/atomisation due to contact with a high velocity gas stream.

The active ingredient (100) is usually in the form of a liquid or oil, but could be any mobile phase carrying the active ingredient

The bag or pouch (150) is usually rolled into a hollow cylinder (See Fig 2B) around first tube (82) for ease of insertion, and the adjoining second tube (84) and frit (120) are inserted into a canister pre-filled with granular activated carbon (130), first and second tubes (82) and (84) being connected to the valve assembly via connectors (182) and (184) respectively. (The granular carbon is easily displaced to accommodate the rolled-up bag which is now surrounded by the activated carbon granules). The canister (90) is then crimped, and the bag side of the canister is filled with the required quantity of active ingredient (100). The frit side of the valve is then filled with pressurised gas (usually, air, oxygen, nitrogen or carbon dioxide). On actuating the valve, the assembly enables the dispensing carrier gas (140), that is mixed or physically saturated, at least in part, with any active ingredient(s), for example, a fragrance for air freshening applications, a drug, or an insecticide. Where the dispensing gas is air or oxygen it is possible to provide a scented air or oxygen, mild enough to breathe. Filling the bag (150) with a medicinal preparation (such as plant oil or an active therefrom) and using the dispensing gas (140) allows for the use as a medical inhaler, optionally fitted with a dose regulator and spacer.

Example 1

An aluminium canister (90) (173 x 53 mm) with an internal volume of 330 ml was filled with a high activity activated carbon (130) (approximately 120 g) and dry ice (140) (57.5 g). The canister was shaken to distribute the mix. A bag- and frit-on-valve assembly (10) as per the first aspect of the invention was taken and 20 ml of pure fragrance oil (100) was added to the bag (150). The bag was inserted into the canister (90) by manipulating it through the activated carbon granules and the canister was crimped to the valve assembly (10) to form a dispenser (20).

The dispenser (20) and its contents was allowed to warm to room temperature. The quantity of carbon dioxide generated a pressure of 12 barg. (Without the activated carbon, it was calculated that the pressure of carbon dioxide in this volume would be equivalent to 54 barg, corresponding to 31 litres of gas).

When the dispenser (20) was actuated an almost dry spray was generated producing a strong and persistent odour. Because the device does not require a solvent, the fragrance is in a concentrated form, and there is no need to identify a compatible solvent. The actuator (200) can be in any design that permits the required amount of scent (or other active component) to be delivered.

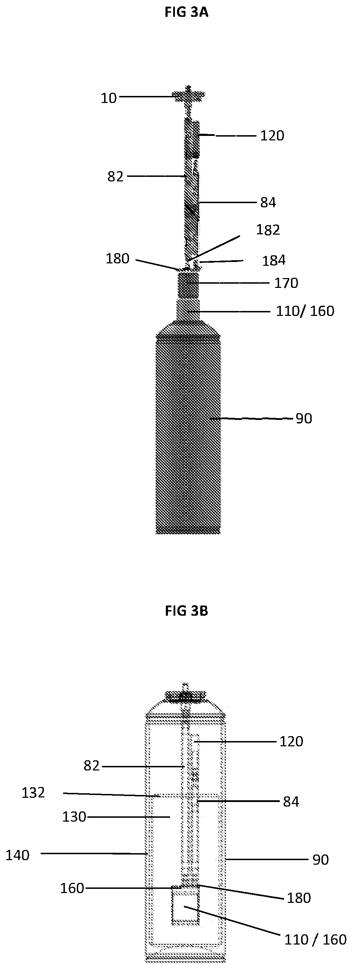

In an alternative design falling outside the scope of the claimed invention (Fig 3A and Fig 3B) the bag (150) is replaced with an ingredient containing container (160), and optionally an absorbent material (170) and closed with a lid (180) comprising two connectors (182) and (184), which connect to first tube (82) and second tube (84), respectively.

The container (160) is connected to the valve assembly (20) via a long tube (82), such that the container (160) is disposed towards the base (92) of the cannister (90). A second tube (84), with a frit (120) on its end, which is ideally, but not essentially, seated above the carbon (130) fill line (132), allows the dispensing gas (140) to pass along tube (84) and into container (160) where it carries ingredient (110) along tube (82) into the valve assembly (10) such that it can leave the cannister (90) *via* the actuator spray nozzle (200). The container (160) may include an absorbent pad (170) that is soaked in the liquid ingredient (110) of choice. Alternatively, the container (160) may house a sublimable solid, such as menthol crystals or camphor. The container (160) and tubes (82; 84) are inserted into the canister (90), connected to the valve assembly (10), pre-filled with granular activated carbon (130) and the canister (90) is gassed under the mounting cup (30) with the aerial gas (140) (preferably, but not limited to, carbon dioxide). The mounting cup (30) is then crimped onto the canister (90). On actuation of the valve, the assembly allows for the saturation of the gas with the fragrance or other ingredient, which is then dispensed into the room or to a subject, without the accompaniment of either solvent or liquefied propellant.

Although this assembly allows for the vapour of the fragrance or other ingredient to diffuse and potentially contact the activated carbon the following example shows that the diffusion of e.g. limonene is very much limited: -

Example 2

Using limonene (molecular mass = 136.2 g/mole, boiling point = 176 °C) as an example, for which the saturation vapour pressure at 25 °C is 2 mm Hg.

At an average can pressure of 5 atmospheres (5.07 bar), the concentration of limonene vapour is: -

$760 \times 2/5 = 5.3E{-}4 \Rightarrow 5.3E{-}4 \times 136.2$ g/mol = 0.072 g limonene/mol gas = 0.072/24 = 3.0E-3 g limonene/litre of gas = 3 g limonene/m$^3$ of gas.

Applying Fick's law: $F = D\,A\,\Delta c/L$

Where: F = diffusion flow rate, D = diffusion coefficient, A = tube cross sectional area (diameter = 4 mm), c = vapour concentration, L = tube length (10 cm).

Hence, $F = 1 \times 10^{-7}$ m$^2$s$^{-1}$ $\times$ 12.6 $\times$ 10$^{-6}$ m$^2$ $\times$ 3 g/m$^3$ $\times$ 1/0.1 m = 3.6 $\times$ 10$^{-11}$ g/s = 0.001 g/year.

The diffusion rate of the limonene onto the activated carbon under these conditions is therefore only 0.001 g/year.

## Claims

1. A dispenser (20) comprising a dispenser container (90) and a valve assembly (10) comprising either a male or female valve connected or crimped to the dispenser container wherein:

   a) the dispenser container (90) is partially filled with activated carbon (130) or another adsorbent and a dispensing carrier gas (140),
   b) an ingredient (100) is contained in an ingredient containing reservoir (110/150), and
   c) the valve assembly (10) comprises a component comprising a dip tube (80), an upper end of which is in operative communication with an actuator spray nozzle (200), and a lower end of which divides into first and second fitments (182; 184) which receive respectively first and second tubes (82; 84); the first tube (82), seated within the dispenser, is connected to the ingredient (100) containing reservoir (110/150), allowing the ingredient to be dispensed on actuation of the valve, and the second tube (84) comprises a frit or filter (120) to prevent the activated carbon (130) or another adsorbent passing into the second tube (84) when the pressurised dispensing carrier gas (140) is released;

   such that on actuation, the dispensing carrier gas (140) is released together with the ingredient (100), and the ingredient (100) and dispensing carrier gas (140) mix as they pass through the valve assembly (10) to exit the dispenser container via the actuator spray nozzle (200).

2. A dispenser as claimed in claim 1 wherein the ingredient containing reservoir (110/150) is operatively connected to the dispenser container (90) such that on actuation of the valve assembly (10) the ingredient (100) and dispensing carrier gas (140) travel along the first tube (82) and the second tube (84) respectively, and mix in the valve assembly (10) before exiting the dispenser container (90) via the actuator spray nozzle (200) to an environment or subject.

3. A dispenser as claimed in claim 1 or 2 wherein the ingredient containing reservoir (110) is a bag or pouch (150).

4. A dispenser as claimed in any of the preceding claims comprising three or more tubes, in addition to the dip tube, and at least two ingredient containing reservoirs (110) comprising different ingredients (100).

5. A dispenser as claimed in any of the preceding claims further comprising a metering device.

6. A dispenser as claimed in claim 5 further comprising a spacer.

7. A dispenser as claimed in any of the preceding claims wherein the dispensing carrier gas is nitrous oxide or an aerial gas, such as air, nitrogen, oxygen, carbon dioxide or argon.

8. A dispenser as claimed in claim 7 wherein the aerial gas is carbon dioxide.

9. A dispenser as claimed in any of the preceding claims which is absent of a liquified propellant and/ or a solvent.

10. A dispenser as claimed in any of the preceding claims wherein the active ingredient is a fragrance, flavour, pheromone, pesticide, nutraceutical or pharmaceutical.

11. A method of delivering an ingredient (100) from a dispenser (20) as claimed in claim 1 wherein the dispensing carrier gas (140) is released together with the ingredient (100), and the ingredient (100) and dispensing carrier gas (140) mix as they pass through the valve assembly (10) to exit the dispenser container via the actuator spray nozzle (200).

12. A method of delivering an ingredient (100) from a dispenser (20) as claimed in claim 11 wherein the dispenser is as claimed in claim 2 wherein the ingredient (100) is released from the ingredient containing reservoir (110) under pressure together with the dispensing carrier gas (140) which is also released on actuation of the valve assembly (10) which ingredient (100) and dispensing carrier gas (140) travel along the first tube (82) and the second tube (84) respectively and mix in the valve assembly (10) before exiting the dispenser container (90) via the actuator spray nozzle (200) to an environment or subject.

**Patentansprüche**

1. Abgabevorrichtung (20), umfassend einen Abgabevorrichtungsbehälter (90) und eine Ventilanordnung (10), die entweder ein männliches oder ein weibliches Ventil umfasst, das mit dem Abgabevorrichtungsbehälter verbunden oder gecrimpt ist, wobei:

   a) der Abgabevorrichtungsbehälter (90) teilweise mit Aktivkohle (130) oder einem anderen Adsorptionsmittel und einem Abgabeträgergas (140) gefüllt ist,
   b) ein Bestandteil (100) in einem einen Bestandteil enthaltenden Reservoir (110/150) enthalten ist und
   c) die Ventilanordnung (10) eine Komponente umfasst, die ein Tauchrohr (80) umfasst, von dem ein oberes Ende mit einer Aktorsprühdüse (200) in Wirkverbindung steht und sich ein unteres Ende in ein erstes und ein zweites Passstück (182; 184) teilt, die jeweils ein erstes und ein zweites Rohr (82; 84) aufnehmen; das erste Rohr (82), das innerhalb der Abgabevorrichtung platziert ist, mit dem einen Bestandteil (100) enthaltenden Reservoir (110/150) verbunden ist, wodurch der Bestandteil bei Betätigung des Ventils abgegeben werden kann, und das zweite Rohr (84) eine Fritte oder einen Filter (120) umfasst, um zu verhindern, dass die Aktivkohle (130) oder ein anderes Adsorptionsmittel in das zweite Rohr (84) gelangt, wenn das unter Druck stehende Abgabeträgergas (140) freigesetzt wird;

   sodass bei Betätigung das Abgabeträgergas (140) zusammen mit dem Bestandteil (100) freigesetzt wird und sich der Bestandteil (100) und das Abgabeträgergas (140) mischen, wenn sie durch die Ventilanordnung (10) gelangen, um den Abgabevorrichtungsbehälter über die Aktorsprühdüse (200) zu verlassen.

**EP 4 234 096 B1**

**2.** Abgabevorrichtung nach Anspruch 1, wobei das einen Bestandteil enthaltende Reservoir (110/150) mit dem Abgabevorrichtungsbehälter (90) wirkverbunden ist, sodass bei Betätigung der Ventilanordnung (10) sich der Bestandteil (100) und das Abgabeträgergas (140) entlang des ersten Rohrs (82) bzw. des zweiten Rohrs (84) bewegen und sich in der Ventilanordnung (10) vermischen, bevor sie den Abgabevorrichtungsbehälter (90) über die Aktorsprühdüse (200) in eine Umgebung oder ein Subjekt verlassen.

**3.** Abgabevorrichtung nach Anspruch 1 oder 2, wobei das einen Bestandteil enthaltende Reservoir (110) ein Beutel oder eine Tasche (150) ist.

**4.** Abgabevorrichtung nach einem der vorhergehenden Ansprüche, umfassend, zusätzlich zu dem Tauchrohr, drei oder mehr Rohre und mindestens zwei ein Bestandteil enthaltendes Reservoire (110), die verschiedene Bestandteile (100) umfassen.

**5.** Abgabevorrichtung nach einem der vorhergehenden Ansprüche, ferner umfassend eine Dosiervorrichtung.

**6.** Abgabevorrichtung nach Anspruch 5, ferner umfassend einen Abstandshalter.

**7.** Abgabevorrichtung nach einem der vorhergehenden Ansprüche, wobei das Abgabeträgergas Lachgas oder ein Luftgas, wie etwa Luft, Stickstoff, Sauerstoff, Kohlendioxid oder Argon, ist.

**8.** Abgabevorrichtung nach Anspruch 7, wobei das Luftgas Kohlendioxid ist.

**9.** Abgabevorrichtung nach einem der vorhergehenden Ansprüche, der kein verflüssigtes Treibmittel und/oder kein Lösungsmittel aufweist.

**10.** Abgabevorrichtung nach einem der vorhergehenden Ansprüche, wobei der aktive Bestandteil ein Duftstoff, Aroma, Pheromon, Pestizid, Nutraceutical oder Pharmazeutikum ist.

**11.** Verfahren zum Abgeben eines Bestandteils (100) aus einer Abgabevorrichtung (20) nach Anspruch 1, wobei das Abgabeträgergas (140) zusammen mit dem Bestandteil (100) freigesetzt wird und sich der Bestandteil (100) und das Abgabeträgergas (140) vermischen, wenn sie durch die Ventilanordnung (10) gelangen, um den Abgabevorrichtungsbehälter über die Aktorsprühdüse (200) zu verlassen.

**12.** Verfahren zum Abgeben eines Bestandteils (100) aus einer Abgabevorrichtung (20) nach Anspruch 11, wobei die Abgabevorrichtung wie in Anspruch 2 beansprucht ist, wobei der Bestandteil (100) aus dem einen Bestandteil enthaltenden Reservoir (110) unter Druck zusammen mit dem Abgabeträgergas (140) freigesetzt wird, das auch bei Betätigung der Ventilanordnung (10) freigesetzt wird, wobei sich der Bestandteil (100) und das Abgabeträgergas (140) entlang des ersten Rohrs (82) bzw. des zweiten Rohrs (84) bewegen und sich in der Ventilanordnung (10) vermischen, bevor sie den Abgabevorrichtungsbehälter (90) über die Aktorsprühdüse (200) in eine Umgebung oder ein Subjekt verlassen.

**Revendications**

**1.** Distributeur (20) comprenant un récipient distributeur (90) et un ensemble vanne (10) comprenant une vanne soit mâle soit femelle connectée ou sertie au récipient distributeur dans lequel :

a) le récipient distributeur (90) est partiellement rempli de charbon actif (130) ou d'un autre adsorbant, ainsi que d'un gaz porteur de distribution (140),
b) un ingrédient (100) est contenu dans un réservoir de contenance d'ingrédient (110/150), et
c) l'ensemble vanne (10) comprend un composant comprenant un tube plongeur (80), dont une extrémité supérieure est en communication opérationnelle avec une buse de pulvérisation d'actionneur (200), et dont une extrémité inférieure se divise en premier et second raccords (182 ; 184) qui reçoivent respectivement des premier et second tubes (82 ; 84) ; le premier tube (82), assis à l'intérieur du distributeur, est raccordé au réservoir (110/150) contenant l'ingrédient (100), permettant à l'ingrédient d'être distribué lors de l'actionnement de la vanne, et le second tube (84) comprend une fritte ou un filtre (120) pour empêcher le charbon actif (130) ou un autre adsorbant de passer dans le second tube (84) lorsque le gaz porteur de distribution sous pression (140) est libéré ;

de sorte que lors de l'actionnement, le gaz porteur de distribution (140) soit libéré conjointement à l'ingrédient (100), et l'ingrédient (100) et le gaz porteur de distribution (140) se mélangent lorsqu'ils passent à travers l'ensemble vanne (10) pour sortir du récipient distributeur via la buse de pulvérisation d'actionneur (200).

2. Distributeur selon la revendication 1, dans lequel le réservoir de contenance d'ingrédient (110/150) est raccordé de manière opérationnelle au récipient distributeur (90) de sorte que lors de l'actionnement de l'ensemble vanne (10), l'ingrédient (100) et le gaz porteur de distribution (140) se déplacent le long du premier tube (82) et du second tube (84) respectivement, et se mélangent dans l'ensemble vanne (10) avant de sortir du récipient distributeur (90) via la buse de pulvérisation d'actionneur (200) vers un environnement ou un sujet.

3. Distributeur selon la revendication 1 ou 2, dans lequel le réservoir de contenance d'ingrédient (110) est un sac ou une poche (150).

4. Distributeur selon l'une quelconque des revendications précédentes, comprenant trois tubes, ou plus, en plus du tube plongeur, et au moins deux réservoirs de contenance d'ingrédient (110) comprenant différents ingrédients (100).

5. Distributeur selon l'une quelconque des revendications précédentes comprenant en outre un dispositif de dosage.

6. Distributeur selon la revendication 5, comprenant en outre une entretoise.

7. Distributeur selon l'une quelconque des revendications précédentes, dans lequel le gaz porteur de distribution est l'oxyde nitreux ou un gaz aérien, tel que l'air, l'azote, l'oxygène, le dioxyde de carbone ou l'argon.

8. Distributeur selon la revendication 7, dans lequel le gaz aérien est le dioxyde de carbone.

9. Distributeur selon l'une quelconque des revendications précédentes, qui est dépourvu de propulseur liquéfié et/ou de solvant.

10. Distributeur selon l'une quelconque des revendications précédentes, dans lequel l'ingrédient actif est un parfum, un arôme, une phéromone, un pesticide, un nutraceutique ou un produit pharmaceutique.

11. Procédé de distribution d'un ingrédient (100) à partir d'un distributeur (20) selon la revendication 1, dans lequel le gaz porteur de distribution (140) est libéré conjointement à l'ingrédient (100), et l'ingrédient (100) et le gaz porteur de distribution (140) se mélangent lorsqu'ils passent à travers l'ensemble vanne (10) pour sortir du récipient distributeur via la buse de pulvérisation d'actionneur (200).

12. Procédé de distribution d'un ingrédient (100) à partir d'un distributeur (20) selon la revendication 11, dans lequel le distributeur est tel que revendiqué dans la revendication 2, dans lequel l'ingrédient (100) est libéré du réservoir de contenance d'ingrédient (110) sous pression avec le gaz porteur de distribution (140) qui est également libéré lors de l'actionnement de l'ensemble vanne (10), lequel ingrédient (100) et lequel gaz porteur de distribution (140) se déplacent le long du premier tube (82) et du second tube (84) respectivement et se mélangent dans l'ensemble vanne (10) avant de sortir du récipient distributeur (90) via la buse de pulvérisation d'actionneur (200) vers un environnement ou un sujet.

FIG 1A

**FIG 1B**

**FIG 2A**

FIG 2B

FIG 2C

**FIG 2D**

**FIG 2E**

FIG 3A

FIG 3B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1703286 A
- WO 2014037086 A
- DE 1817899
- DE 1817840 A1